# EUROPÄISCHE PATENTSCHRIFT

(11) **EP 0 708 176 B1**
(45) Veröffentlichungstag und Bekanntmachung des Hinweises auf die Patenterteilung: **06.10.2004**
(21) Anmeldenummer: 95116245.2
(22) Anmeldetag: 16.10.1995
(51) Int. Cl.: C12N 1/10

(54) **Verfahren zur Kultivierung lipidabhängiger Tetrahymeniden und ein Verfahren zur Herstellung von biogenen Wertstoffen aus lipidabhängigen Tetrahymeniden**
Process for the cultivation of lipid-dependent tetrohymenids and a process for the production of biogenic products of lipid-dependent tetrahymenids
Procédé de culture de tétrahyménidies et utilisation de substances produites par ces tétrahyménidies

(30) Priorität: 21.10.1994 DE 4437615
(43) Veröffentlichungstag der Anmeldung: 24.04.1996
(73) Patentinhaber: Nutrinova Nutrition Specialties & Food Ingredients GmbH, 65926 Frankfurt am Main (DE)
(72) Erfinder: Kiy, Thomas, Dr., D-65929 Frankfurt (DE); Marquardt, Rüdiger, Dr., D-60389 Frankfurt (DE); Kühlein, Klaus, Dr., D-65779 Kelkheim (DE)
(74) Vertreter: Luderschmidt, Schüler & Partner

(56) Entgegenhaltungen:
- WO-A-86/03518
- DE-A- 4 238 842
- DOBRA K W ET AL: "GROWTH KINETICS OF THREE SPECIES OF TETRAHYMENA ON SOLID AGAR" JOURNAL OF PROTOZOOLOGY, Bd. 27, Nr. 2, 1. Mai 1980, Seiten 226-230, XP000561009
- LUKSAS A J ET AL: "THE LIPID REQUIREMENT OF THE CILIATED PROTOZOON TETRAHYMENA PATULA" JOURNAL OF PROTOZOOLOGY, Bd. 30, Nr. 1, 1. Januar 1983, Seiten 8-13, XP000561012
- GOSSELIN Y ET AL: "IMPROVEMENT OF FED BATCH MASS CULTURE FOR LINOLENIC BIOSYNTHESIS BY TETRABYMENA ROSTRATA (PROTOZOA)" BIOTECHNOLOGY LETTERS, Bd. 11, Nr. 6, 1. Juni 1989, Seiten 423-426, XP000561016
- KIY T ET AL: "MASS CULTIVATION OF TETRAHYMENA THERMOPHILA YIELDING HIGH CELL DENSITIES AND SHORT GENERATION TIMES" APPLIED MICROBIOLOGY AND BIOTECHNOLOGY, Bd. 37, Nr. 5, 1. August 1992, Seiten 576-579, XP000561042
- KIY T ET AL: "CONTINUOUS HIGH-CELL-DENSITY FERMENTATION OF THE CILIATED PROTOZOONTETRAHYMENA IN A PERFUSED BIOREACTOR" APPLIED MICROBIOLOGY AND BIOTECHNOLOGY, Bd. 38, Nr. 2, 1. November 1992, Seiten 141-146, XP000561041
- DATABASE BIOSIS, BIOSCIENCES INFORMATION SERVICE, PHILADELPHIA, PA, US, BIOSIS Nr. PREV199396124805, CHRISTENSEN SOREN T ET AL: "Cell multiplicaiton in Tetrahymena setosa and Tetrahymena thermophila in synthetic nutrient medium: Effects of ethanol, cholesterol and extracellular medium", 1993

## Beschreibung

Die vorliegende Erfindung betrifft ein Verfahren zur Kultivierung von lipidabhängigen Tetrahymeniden, sogenannten lipid requirer oder fastidious species, insbesondere von Tetrahymena setosa, sowie ein Verfahren zur Herstellung von biogenen Wertstoffen aus lipidabhängigen Tetrahymeniden, insbesondere von Enzymen welche von den lipidabhängigen Tetrahymeniden in das umgebende Medium sezerniert werden, von solchen Enzymen, welche zellassoziiert vorliegen und von mehrfach ungesättigten Fettsäuren.

Bereits 1972 wurde gezeigt, daß das holotriche Ciliat Tetrahymena pyriformis die lysosomalen Enzyme β-N-Acetylhexosaminidase, saure Phosphatase, α- und β-Glucosidase, Amylase, Proteinase, Desoxyribonuclease und Ribonuclease ins umgebende Medium sezerniert (M. Müller, 1972, J. Cell Biol., 52, 478). Blum (J. J. Blum, 1975, J. Cell. Physiol., 86, 131) konnte zusätzlich die Sekretion von α-Mannosidase, β-Fucosidase und β-Galactosidase nachweisen. Im Fall von T. thermophila (ehemals Paarungstyp 1 von T. pyriformis, daher also sehr eng verwandt) konnte die Sekretion von β-N-Acetylhexosaminidase, saure Phosphatase, α- und β-Glucosidase, α-Mannosidase, Protease und Phosphodiesterase I gezeigt werden (T. Kiy & A. Tiedtke, 1991, Appl. Microbiol. Biotechnol., 35, 18; T. Kiy & A. Tiedtke, 1993, BioEng., 5, 22); Phospholipase C und Phospholipase A₁ wurden ebenfalls als sekretorische Enzyme von T. thermophila identifiziert (J. Florin-Christensen et al., 1986, Comp. Biochem. Physiol., 85B, 149; J. Florin-Christensen et al., 1986, Comp. Biochem. Physiol., 85B, 143).

Die Verwendungsmöglichkeiten dieser von den genannten Arten produzierten Enzyme sind vielfältig:
β-Galactosidasen können z. B. zur Spaltung des Milchzuckers (Lactose) und bei der β-Galactosidase-katalysierten Transglycosidierung - z. B. zur Herstellung von β-Galacto-, Arabino- und Fucopyranosiden - verwendet werden.
Die Phosphatasen können beispielsweise zur Reduzierung des Phosphatgehalts von Lebensmitteln eingesetzt werden.
β-Glucosidasen lassen sich zur Synthese von Glucosacchariden verwenden.
Die Proteasen können unter Ausnutzung der Acyltransferasereaktion zur Peptidsynthese verwendet werden.

Neben zellassoziierten Enzymen und Enzymen, die von diesen Arten in das umgebende Medium sezerniert werden, können aus den Kulturüberständen oder aus den Zellen selbst weitere Wertstoffe von ökonomischen Interesse durch die Kultivierung dieser Ciliaten gewonnen werden. So wurden aus T. termophila-Zellen γ-Linolensäure und aus dem Kulturüberstand von T. thermophila-Pigment-Mutanten Melanin-Vorstufen isoliert (T. Kiy & A. Tiedtke, 1993, BioEng., 5, 22).

Die beiden eingangs genannten Arten gehören zum sogenannten T. pyriformis-Komplex und sind relativ einfach auf simplen, preiswerten Nährmedien zu züchten. Daher sind auch einfache axenische Kulturmedien für eine Massenkultivierung der Arten des Pyriformis-Komplexes geeignet (K. Keenan et al., 1978, J. Protozool., 25, 385).
Als Kulturmedien für die Massenkultivierung dieser Vertreter des Pyriformis-Komplexes werden unter anderem das sogenannte TGMM-2 Medium (K. Keenan et al., 1978, J. Protozool., 25, 385), bestehend pro 100 ml Wasser aus 0,3 g Bierhefe, 1 mg Soja-Lecithin sowie 0,1 mg Magermilchpulver, und neuerdings ein sogenanntes Magermilchmedium (T. Kiy & A. Tiedtke, 1992, Appl. Microbiol. Biotechnol., 37, 576), bestehend aus Magermilchpulver, Hefeextrakt und Glucose, vorgeschlagen.

Erst durch die Massenkultivierung aber wurden Nachweis und Isolierung der von diesen Mikroorganismen produzierten Naturstoffe ermöglicht.

Obwohl hydrolytische, lysosomale Enzyme und andere Naturstoffe von ökonomischem Interesse sind, wurde es bis heute versäumt, unter den ca. 8000 verschiedenen Ciliatenarten nach weiteren und gegebenenfalls besseren Enzymproduzenten und Enzymen zu suchen.

Allerdings ist es bislang nicht gelungen, Ciliaten aus der Gruppe der lipidabhängigen Tetrahymeniden, der sogenannten "lipid-requirer" oder "fastidious species" (z. B. T. setosa, T. rostrata, T. paravorax, T. patula u. a.) über längere Zeit und in Massen zu kultivieren. Diese Ciliaten sind in ihrem Wachstum sehr abhängig und weisen besondere Nahrungsansprüche auf. Ihr Potential ist daher weitgehend unerforscht (K. Keenan et al., 1978, J. Protozool., 25, 385). Infolgedessen liegen auch keine Untersuchungen hinsichtlich des Vorhandenseins wertvoller Substanzen - etwa Enzyme - vor.

Demgegenüber ist es Aufgabe der vorliegenden Erfindung, ein Verfahren zur Kultivierung von lipidabhängigen Tetrahymeniden (lipid requirer) bereitzustellen, das geeignet ist, lipidabhängige Tetrahymeniden (lipid requirer) in hohen Zelldichten und über längere Zeit in Massen auf einfachen Kulturmedien zu halten, woraus sezernierte oder zellassoziierte, biogene Wertstoffe, insbesondere Enzyme und ungesättigte Fettsäuren, gewonnen werden können.

Überraschenderweise wurde gefunden, daß unter bestimmten Voraussetzungen sich einfache Medien, denen keine Lipide zugesetzt worden sind, besonders gut zur Massenkultivierung von lipidabhängigen Tetrahymeniden (lipid requirer) eignen.

Die Aufgabe wird dementsprechend erfindungsgemäß gelöst durch ein Verfahren zur Kultivierung lipidabhängiger Tetrahymeniden (lipid requirer), welches sich dadurch auszeichnet, daß man die lipidabhängigen Tetrahymeniden auf einem Medium kultiviert, das
5 bis 100 g/l Magermilchpulver oder Baumwollsamenmehl,
1 bis 20 g/l Hefeextrakt,
0,03 g/l Eisen in Form komplexierter Eisenionen und
0 bis 100 g/l Glucose enthält.

Vorzugsweise enthält das Medium
20 g/l Magermilchpulver,
5 g/l Hefeextrakt,
0,03 g/l Eisen in Form komplexierter Eisenionen und
10 g/l Glucose.

Eine weitere bevorzugte Ausgestaltung dieses Verfahrens ist, daß das
Kulturmedium
20 g/l Baumwollsamenmehl,
5 g/l Hefeextrakt,
0,03 g/l Eisen in Form komplexierter Eisenionen und
10 g/l Glucose enthält. Wahlweise kann bei dieser Zusammensetzung dem Medium noch 10 ml/l Sojaöl zugesetzt werden.

Als komplexierte Eisenionen sind Eisenionen in Form des Eisen-Ethylendiamintetraacetat-Komplexes (EDTA-Komplex) geeignet.

Das Eisen kann dem Kulturmedium auch in Form von Eisencitrat zugesetzt werden.

Unter den genannten Bedingungen kann die Kultivierung auch unter Zellrückhaltung mittels Membranen kontinuierlich erfolgen.

Als lipidabhängiges Tetrahymenid wird bevorzugt Tetrahymena rostrata, Tetrahymena patula oder Tetrahymena setosa, besonders bevorzugt Tetrahymena rostrata ATCC 30770, Tetrahymena patula CCAP 1630/2 oder Tetrahymena setosa ATCC 30782, deren Mutanten oder deren Varianten eingesetzt.

Die eingangs gestellte Aufgabe wird ferner gelöst durch ein Verfahren zur Herstellung von biogenen Wertstoffen aus lipidabhängigen Tetrahymeniden (lipid requirer), dadurch gekennzeichnet, daß man die lipidabhängigen Tetrahymeniden mittels des oben beschriebenen Verfahrens kultiviert und die biogenen Wertstoffe nach bekannten Methoden isoliert.

Enzyme, welche von lipidabhängigen Tetrahymeniden (lipid requirer) in das umgebende Medium sezerniert werden, lassen sich vorzugsweise dadurch herstellen, daß man die lipidabhängigen Tetrahymeniden mittels des oben beschriebenen Verfahrens kultiviert, bis sich die sezernierten Enzyme im Kulturmedium angereichert haben, und man anschließend die Enzyme nach bekannten Verfahren aus dem zellfreien Kulturüberstand isoliert und durch chromatographische Verfahren voneinander trennt.

Zellassoziierte Enzyme aus lipidabhängigen Tetrahymeniden (lipid requirer) werden vorzugsweise dadurch gewonnen, daß man die lipidabhängigen Tetrahymeniden mittels des oben beschriebenen Verfahrens kultiviert, die Zellen homogenisiert, die enzymhaltige Lösung mittels Zentrifugation oder Filtration von den aufgeschlossenen Zellen abtrennt und die Enzyme durch chromatographische Verfahren voneinander trennt.

Ferner lassen sich durch das Verfahren insbesondere auch mehrfach ungesättigte Fettsäuren gewinnen.

Im folgenden wird die Erfindung im einzelnen beschrieben und durch Beispiele näher erläutert.

Mit dem Verfahren gemäß der vorliegenden Erfindung können lipidabhängige Tetrahymeniden (lipid requirer), wie T. setosa, T. rostrata oder T. patula, erfolgreich in hohen Zelldichten auf einfachen Komplexmedien kultiviert werden. Dadurch ist es möglich, lipidabhängige Tetrahymeniden auf das Vorhandensein von insbesondere hydrolytischen, lysosomalen Enzymen zu untersuchen. Im zellfreien Überstand und im Zellhomogenat dieser Kulturen können beispielsweise folgende Enzymaktivitäten nachgewiesen werden: Alkalische Phosphatase, Esterase (C4), Esterase Lipase (C8), Phosphodiesterase, Arylamidase, Trypsin, Chymotrypsin, saure Phosphatase, Naphthol-AS-BI-Phosphohydrolase, β-Galactosidase, α- und β-Glucosidase, N-Acetyl-β-Glucosaminidase, α- und β-Mannosidase und β-Fucosidase. Die Vorteile dieser Enzyme sind, daß sie im Vergleich zu den meisten lysosomalen, aus anderen Quellen stammenden Enzymen sezerniert werden und auch außerhalb der Zellen sehr stabil sind.

Tetrahymena patula CCAP 1630/2 wurde von der Culture Collection of Algae and Protozoa, Freshwater Biological Association, The Ferry House, Ambleside, Cumbria LA220LP, U. K., Tetrahymena setosa ATCC 30782 und Tetrahymena rostrata ATCC 30770 wurden von der American Type Culture Collection (ATCC), 12301 Parklawn Drive, Rockville, Maryland, USA, bezogen. Anstelle von T. patula CCAP 1630/2, T. setosa ATCC 30782 und T. rostrata ATCC 30770 können auch deren Mutanten und Varianten verwendet werden, soweit sie die genannten enzymatischen Aktivitäten weiterhin aufweisen. Solche Mutanten lassen sich z. B. mittels der für T. thermophila beschriebenen Methode (P. Hünseler et al., 1987, J. Cell Sci., 88, 47) unter Einsatz von N-Methyl-N'-nitro-N'-nitrosoguanidin gewinnen.

T. setosa wurde gemäß dem Verfahren der vorliegenden Erfindung auf Magermilchmedium und auf Pharmamedia®-Medium, bestehend im wesentlichen aus Baumwollsamenmehl, erstmals erfolgreich in Massen kultiviert. Dabei zeigte sich, daß das Wachstum auf diesen Medien verglichen mit dem bisher bekanntem TGMM-2-Medium deutlich besser ausfällt.

Wird T. setosa auf dem bisher verwendeten TGMM-2-Medium (American Type Culture Collection Catalogue, Algae & Protozoa, 18th edition, 1993, Medium 986) in Batch-Kultur gezüchtet (Schüttelkultur, 25°C), so erreicht man nach 28 h eine Zelldichte von 155.000 Zellen/ml.

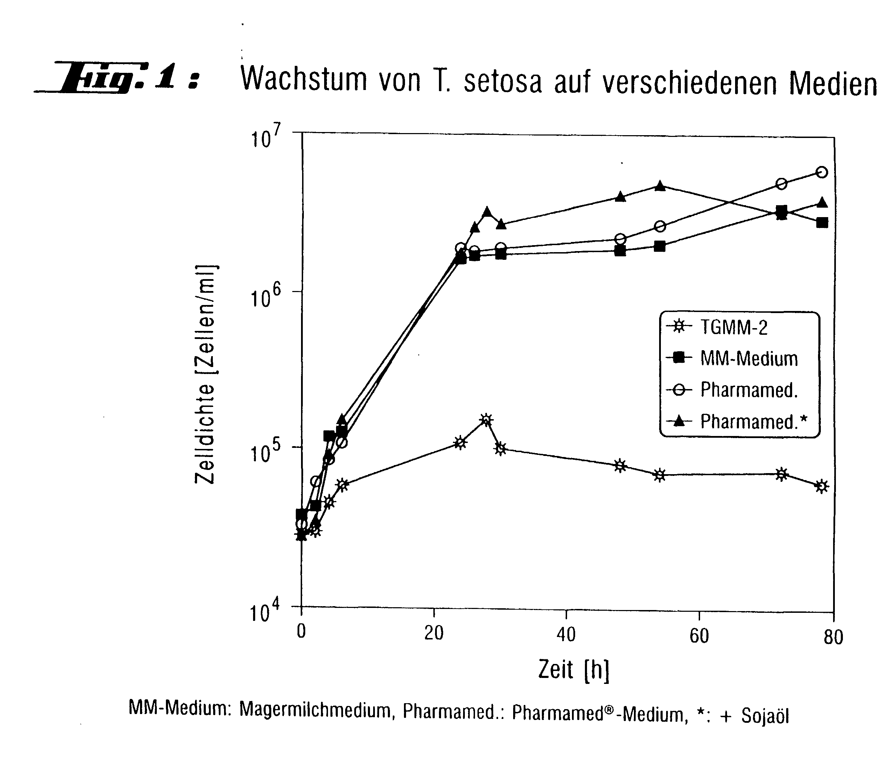

Unter gleichen Bedingungen ist die Zelldichte 23mal höher in Magermilchmedium (3.500.000 Zellen/ml), 39mal höher in Pharmamedia ohne Sojaöl (6.000.000 Zellen/ml) und 32mal höher in Pharmamedia + Sojaöl (5.000.000 Zellen/ml) (Fig.1).

Gleiche Ergebnisse lassen sich auch mit T. rostrata und T. patula erzielen. In diesem Zusammenhang erwies sich überraschenderweise, daß sich Vertreter der Gruppe der lipidabhängigen Tetrahymeniden (lipid-requirer) hervorragend als Enzymproduzenten eignen. So wurden beispielsweise in einer T. pyriformis-Kultur nach 80 h ca. 210 mU β-Hexosaminidase und 750 mU saure Phosphatase nachgewiesen (Y. Banno et al., 1987, Exp. Cell Res., 170, 259). Demgegenüber konnten in dem hier beschriebenen System bereits nach 72 h 800 mU β-Hexosaminidase und > 10.000 mU saure Phosphatase detektiert werden.

Als Nährstoffquellen für T. setosa, T. patula und T. rostrata sind außerdem auch Peptone, Tryptone, Fleischextrakte bzw. -mehle und andere gemahlene Samen (z. B. Mais-, Hafer-, Weizen-, Soja- und Bohnensamen) geeignet. Außerdem kann die Nährlösung Lipidquellen (z. B. Asolectin) enthalten.

Die Zucht der lipidabhängigen Tetrahymeniden (lipid requirer) erfolgt aerob, also beispielsweise submers unter Schütteln oder Rühren in Schüttelkolben oder Fermentern, gegebenenfalls unter Einführung von Luft oder Sauerstoff. Zusätzlich kann eine pH-Regulierung erfolgen. Die Anzucht kann in einem Temperaturbereich von 18 bis 37°C, vorzugsweise aber zwischen 23 und 33°C, erfolgen. Man kultiviert T. setosa unter den genannten Bedingungen, bis die Enzyme sich in der Kultur anhäufen. Alternativ kann die Fermentation, wie anhand von T. thermophila bereits gezeigt (T. Kiy & A. Tiedtke, 1992, Appl. Microbiol. Biotechnol. 38, 141), kontinuierlich durchgeführt werden. In diesem Fall wird das produkthaltige Kulturmedium kontinuierlich geerntet.

Vorzugsweise kultiviert man in mehreren Stufen, d. h. aus einer Stammkultur wird eine Vorkultur, wobei bereits das Produktionsmedium eingesetzt wird, angeimpft. Nach etwa 24 bis 72 h wird mit der Vorkultur die Hauptkultur, beispielsweise im Volumenverhältnis 1 : 10, angeimpft.

Die Fermentation kann durch Bestimmung der Biomasse oder der Zellkonzentration überwacht werden. Die Produktkonzentration läßt sich durch die Bestimmung der Volumenaktivität der Enzyme verfolgen.

Die Isolierung der Enzyme kann entweder aus dem zellfreien Überstand oder aus dem Zellhomogenat erfolgen.

Sollen die Enzyme aus dem zellfreien Kulturüberstand isoliert werden, so müssen die Zellen vom Kulturmedium abgetrennt werden. Dies kann z. B. über Zellimmobilisierung (T. Kiy & A. Tiedtke, 1991, Appl. Microbiol. Biotechnol., 35, 18) oder durch die Verwendung von Membran- bzw. Spinfilterbioreaktoren bereits während der Fermentation geschehen, oder aber über Tangentialfiltration oder Zentrifugation nach Beendigung der Fermentation.

Sollen zusätzlich die zellassoziierten Enzyme gewonnen werden, müssen die Zellen zunächst homogenisiert werden. Besonders geeignet sind in diesem Zusammenhang Ultraschall- bzw. Ultraturraxgeräte, aber auch einfaches Einfrieren reicht aus, um die Zellen aufzuschließen. Über Zentrifugation oder Filtration läßt sich partikuläres Material nach dem Homogenisieren vom Überstand abtrennen.

Die Trennung und Reinigung der Enzyme erfolgt über chromatographische Methoden. Die enzymhaltige Probe läßt sich z. B. zunächst auf eine Anionenaustauschersäule (z. B. Q-Sepharose®, Mono Q) auftragen. Die Elution erfolgt über einen aufsteigenden Salzgradienten. Alternativ kann eine Chromatofokussiersäule (Mono P) eingesetzt werden, wobei mittels eines absteigenden pH-Gradienten eluiert wird. Zur Trennung der Enzyme von kleinmolekularen Substanzen hat sich der Einsatz einer Gelfiltration (z. B. Superose®, Superdex®) als sinnvoll erwiesen.

Zur gezielten Aufreinigung einzelner Enzyme bietet sich der Einsatz von affinitätschromatographischen Medien an. Bevorzugt werden hier Substratanaloga, Inhibitoren oder gegen das Enzym gerichtete spezifische Antikörper als Liganden verwendet.

### Beispiele:

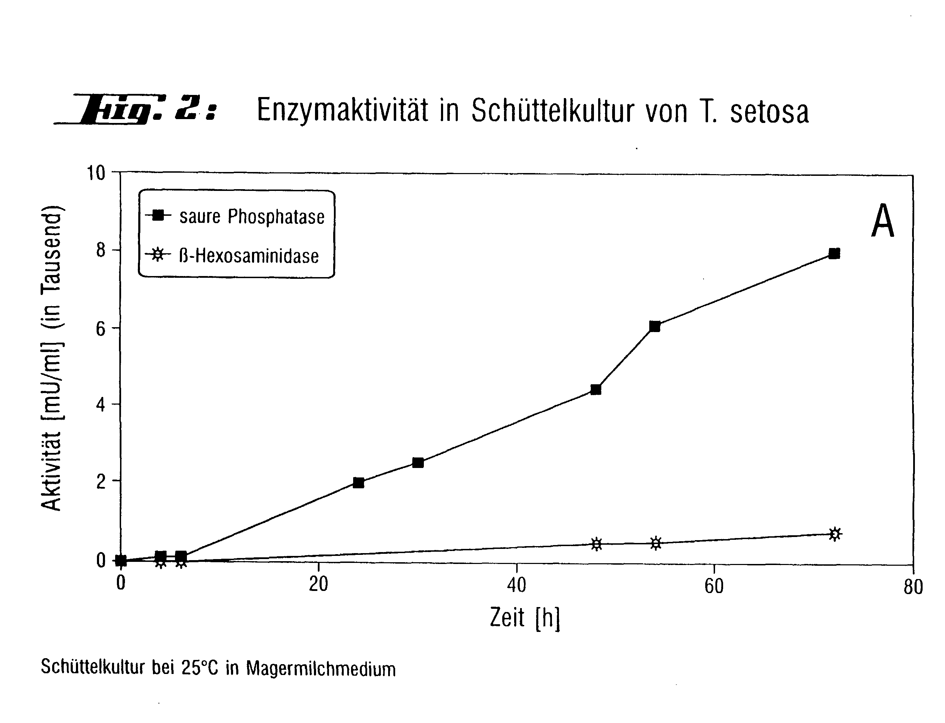

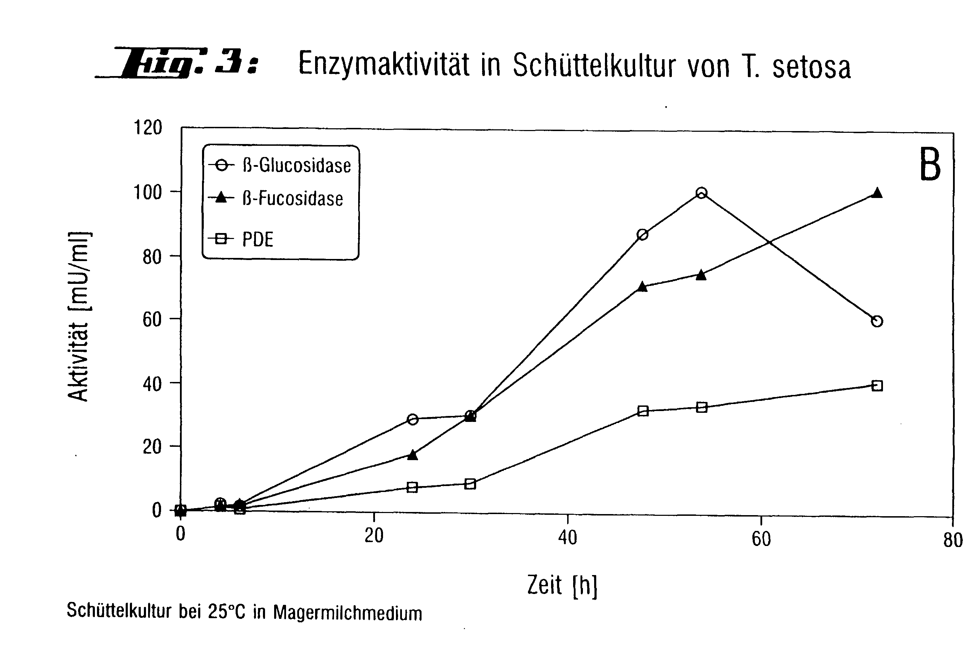

1. Stammkultur des Produzentenstammes
   Die Dauerkultivierung erfolgt bei 20°C in Reagenzgläsern auf autoklavierten Kichererbsen. Dazu wird eine Kichererbse in 10 ml Aqua demin. pro Reagenzglas eingesetzt. Das Umimpfen erfolgt halbjährlich.
2. Vor- bzw. Hauptkultur des Produzentenstammes in Erlenmeyerkolben
   Ein 100-ml-Erlenmeyerkolben, der 10 ml Kulturmedium enthält, wird mit 1 ml der Stammkultur angeimpft und bei 25°C über Nacht auf einem Schüttler (80 rpm) inkubiert. Am nächsten Tag wird ein 500-ml-Erlenmeyerkolben, der 100 ml Kulturmedium enthält, mit 10 ml dieser Vorkultur inokuliert. Nach 24 h werden 500 ml Medium (in 2,5-l-Erlenmeyerkolben) mit 30 ml dieser Vorkultur angeimpft. Die maximale volumetrische Aktivität ist je nach Enzym zwischen 40 und 90 h erreicht. Die Kultur enthält z. B. bis zu 8.000 mU/ml saure Phosphatase, 800 mU/ml β-Hexosaminidase, 100 mU/ml β-Glucosidase, 100 mU/ml β-Fucosidase und 40 mU/ml Phosphodiesterase (Fig. 2 und 3). Wird die Kultivierung kontinuierlich mit Zellrückhaltung - z. B. in einem Membranbioreaktor - durchgeführt, so ist eine Steigerung der volumetrischen Aktivität bis um das 50fache realisierbar.
   Das Produktionsmedium enthält Magermilchpulver (20 g/l), Hefeextrakt (5 g/l), Sequestrene (0,003 %) und Glucose (10 g/l). Die Glucose wird getrennt autoklaviert und erst nach dem Abkühlen dem Kulturmedium zugeführt. Alternativ kann Pharmamedia®-Medium (20 g/l Baumwollsamenmehl, 10 g/l Glucose, 5 g/l Hefeextrakt und 0,003% Sequestrene® oder 1 ml/l Eisenspur) verwendet werden, wobei das Medium zusätzlich mit 10 ml/l Sojaöl supplementiert werden kann.
3. Ernte des enzymhaltigen Kulturmediums
   Die Zellen aus insgesamt 2,5 l Kultur werden vom Medium über eine FILTRON®-Tangentialfiltrationseinheit getrennt (Porengröße der Membran, 0,3 µm). In einem zweiten Schritt wird der zellfreie Überstand durch Verwendung einer anderen Membran (Ausschlußgröße, 10 kDa) bis auf ein Volumen von 300 ml eingeengt. Über eine AMICON®-Zelle (PM 10-Membran) wird das Konzentrat in den Startpuffer für die nachfolgende chromatographische Reinigung umdialysiert und gleichzeitig weiter auf ein Volumen von 50 ml konzentriert.
4. Aufarbeitung des enzymhaltigen Konzentrats
   Die in Puffer A (20 mM Triethanolamin , pH: 7,8) umdialysierte Probe wird auf eine Q-Sepharose®-Säule aufgetragen. Für die Elution wird ein 20 mM Triethanolamin-Puffer (pH: 7,8) eingesetzt, der NaCl (1M) enthält. Während einige der lysosomalen Enzyme im Durchbruch enthalten sind (z. B. β-Galactosidase, β-Fucosidase), werden die anderen im Verlauf des Gradienten eluiert.
   Fraktionen, die das gewünschte Enzym enthalten, werden gepoolt und in Citratpuffer (50 mM Citrat, 100 mM NaCl, pH: 6,5) umdialysiert.
   Über eine Gelfiltration (Superose® 12) werden kleinmolekulare Verunreinigungen von den Enzymen getrennt.

## Patentansprüche

1. Verfahren zur Kultivierung lipidabhängiger Tetrahymeniden (lipid requirer), **dadurch gekennzeichnet, daß** man die lipidabhängigen Tetrahymeniden auf einem Medium kultiviert, das
5 bis 100 g/l Magermilchpulver oder Baumwollsamenmehl,
1 bis 20 g/l Hefeextrakt,
0,03 g/l Eisen in Form komplexierter Eisenionen und
0 bis 100 g/l Glucose enthält.

2. Verfahren nach Anspruch 1, **dadurch gekennzeichnet, daß** das Medium
20 g/l Magermilchpulver,
5 g/l Hefeextrakt,
0,03 g/l Eisen in Form komplexierter Eisenionen und
10 g/l Glucose enthält.

3. Verfahren nach Anspruch 1, **dadurch gekennzeichnet, daß** das Medium
20 g/l Baumwollsamenmehl,
5 g/l Hefeextrakt,
0,03 g/l Eisen in Form komplexierter Eisenionen und
10 g/l Glucose enthält.

4. Verfahren nach Anspruch 3, **dadurch gekennzeichnet, daß** das Medium 10 ml/l Sojaöl enthält.

5. Verfahren nach einem der Ansprüche 1 bis 4, **dadurch gekennzeichnet, daß** die komplexierten Eisenionen als EDTA-Komplex vorliegen.

6. Verfahren nach einem der Ansprüche 1 bis 4, **dadurch gekennzeichnet, daß** das Eisen als Eisencitrat eingesetzt wird.

7. Verfahren nach einem der Ansprüche 1 bis 6, **dadurch gekennzeichnet, daß** die Kultivierung unter Zellrückhaltung mittels Membranen kontinuierlich erfolgt.

8. Verfahren nach einem der Ansprüche 1 bis 7, **dadurch gekennzeichnet, daß** das lipidabhängige Tetrahymenid Tetrahymena setosa ist.

9. Verfahren nach Anspruch 8, **dadurch gekennzeichnet, daß** als lipidabhängige Tetrahymeniden Tetrahymena setosa ATCC 30782, dessen Mutanten oder dessen Varianten verwendet werden.

10. Verfahren nach einem der Ansprüche 1 bis 7, **dadurch gekennzeichnet, daß** das lipidabhängige Tetrahymenid Tetrahymena rostrata ist.

11. Verfahren nach Anspruch 10, **dadurch gekennzeichnet, daß** das lipidabhängige Tetrahymenid Tetrahymena rostrata ATCC 30770 ist.

12. Verfahren nach einem der Ansprüche 1 bis 7, **dadurch gekennzeichnet, daß** das lipidabhängige Tetrahymenid Tetrahymena patula ist.

13. Verfahren nach Anspruch 12, **dadurch gekennzeichnet, daß** das lipidabhängige Tetrahymenid Tetrahymena patula CCAP 1630/2 ist.

14. Verfahren zur Herstellung von biogenen Wertstoffen aus lipidabhängigen Tetrahymeniden (lipid requirer), **dadurch gekennzeichnet, daß** man die lipidabhängigen Tetrahymeniden mittels eines Verfahrens gemäß einem der Ansprüche 1 bis 13 kultiviert und die biogenen Wertstoffe nach bekannten Methoden isoliert.

15. Verfahren nach Anspruch 14 zur Herstellung von Enzymen, welche von lipidabhängigen Tetrahymeniden (lipid requirer) in das umgebende Medium sezerniert werden, **dadurch gekennzeichnet, daß** man die lipidabhängigen Tetrahymeniden mittels eines Verfahrens gemäß einem der Ansprüche 1 bis 13 kultiviert bis sich die sezernierten Enzyme im Kulturmedium angereichert haben und man anschließend die Enzyme nach bekannten Verfahren aus dem zellfreien Kulturüberstand isoliert und durch chromatographische Verfahren voneinander trennt.

16. Verfahren nach Anspruch 14 zur Herstellung von zellassoziierten Enzymen aus lipidabhängigen Tetrahymeniden (lipid requirer), **dadurch gekennzeichnet, daß** man die lipidabhängigen Tetrahymeniden mittels eines Verfahrens gemäß einem der Ansprüche 1 bis 13 kultiviert, die Zellen homogenisiert, die enzymhaltige Lösung mittels Zentrifugation oder Filtration von den aufgeschlossenen Zellen abtrennt und die Enzyme durch chromatographische Verfahren voneinander trennt.

17. Verfahren nach Anspruch 14, **dadurch gekennzeichnet, daß** die biogenen Werstoffe mehrfach ungesättigte Fettsäuren sind.

## Claims

1. A process for the cultivation of lipid-dependent tetrahymenids (lipid requirers), **characterised in that** the lipid-dependent tetrahymenids are cultivated on a medium that comprises
from 5 to 100 g/l skimmed milk powder or cottonseed meal,
from 1 to 20 g/l yeast extract,
0.03 g/l iron in the form of complexed iron ions and
from 0 to 100 g/l glucose.

2. A process according to claim 1, **characterised in that** the medium comprises
20 g/l skimmed milk powder,
5 g/l yeast extract,
0.03 g/l iron in the form of complexed iron ions and
10 g/l glucose.

3. A process according to claim 1, **characterised in that** the medium comprises
20 g/l cottonseed meal,
5 g/l yeast extract,
0.03 g/l iron in the form of complexed iron ions and
10 g/l glucose.

4. A process according to claim 3, **characterised in that** the medium comprises 10 ml/l soybean oil.

5. A process according to any one of claims 1 to 4, **characterised in that** the complexed iron ions are in the form of an EDTA complex.

6. A process according to any one of claims 1 to 4, **characterised in that** the iron is used as iron citrate.

7. A process according to any one of claims 1 to 6, **characterised in that** the cultivation is carried out continuously with the cells being retained by means of membranes.

8. A process according to any one of claims 1 to 7, **characterised in that** the lipid-dependent tetrahymenid is Tetrahymena setosa.

9. A process according to claim 8, **characterised in that** Tetrahymena setosa ATCC 30782, mutants thereof or variants thereof are used as the lipid-dependent tetrahymenids.

10. A process according to any one of claims 1 to 7, **characterised in that** the lipid-dependent tetrahymenid is Tetrahymena rostrata.

11. A process according to claim 10, **characterised in that** the lipid-dependent tetrahymenid is Tetrahymena rostrata ATCC 30770.

12. A process according to any one of claims 1 to 7, **characterised in that** the lipid-dependent tetrahymenid is Tetrahymena patula.

13. A process according to claim 12, **characterised in that** the lipid-dependent tetrahymenid is Tetrahymena patula CCAP 1630/2.

14. A process for the production of biogenous valuable substances from lipid-dependent tetrahymenids (lipid requirers), **characterised in that** the lipid-dependent tetrahymenids are cultivated by means of a process according to any one of claims 1 to 13 and the biogenous valuable substances are isolated by known methods.

15. A process according to claim 14 for the production of enzymes that are secreted by lipid-dependent tetrahymenids (lipid requirers) into the surrounding medium, **characterised in that** the lipid-dependent tetrahymenids are cultivated by means of a process according to any one of claims 1 to 13 until the secreted enzymes have become enriched in the culture medium and then the enzymes are isolated from the cell-free culture supernatant by known methods and separated from one another by chromatographic methods.

16. A process according to claim 14 for the production of cell-associated enzymes from lipid-dependent tetrahymenids (lipid requirers), **characterised in that** the lipid-dependent tetrahymenids are cultivated by means of a process according to any one of claims 1 to 13, the cells are homogenised, the enzyme-containing solution is separated from the lysed cells by means of centrifugation or filtration, and the enzymes are separated from one another by chromatographic methods.

17. A process according to claim 14, **characterised in that** the biogenous valuable substances are polyunsaturated fatty acids.

## Revendications

1. Procédé de culture de Tetrahymenae dépendants de lipides (lipid requirer), **caractérisé en ce qu'**on cultive les *Tetrahymenae* dépendants des lipides sur un milieu qui renferme
- de 5 à 100 g/l de poudre de lait écrémé ou de poudre de grains de coton,
- de 1 à 20 g/l d'extrait de levure,
- 0,03 g/l de fer sous forme d'ions fer complexé et
- de 0 à 100 g/l de glucose.

2. Procédé selon la revendication 1, **caractérisé en ce que** le milieu renferme
- 20 g/l de poudre de lait écrémé,
- 5 g/l d'extrait de levure,
- 0,03 g/l de fer sous forme d'ions fer complexé et
- 10 g/l de glucose.

3. Procédé selon la revendication 1, **caractérisé en ce que** le milieu renferme
- 20 g/l de poudre de grains de coton,
- 5 g/l d'extrait de levure,
- 0,03 g/l de fer sous forme d'ions fer complexé et
- 10 g/l de glucose.

4. Procédé selon la revendication 3, **caractérisé en ce que** le milieu renferme 10 ml/l d'huile de soja.

5. Procédé selon l'une des revendications 1 à 4, **caractérisé en ce que** les ions fer complexés sont présents sous forme de complexe EDTA.

6. Procédé selon l'une des revendications 1 à 4, **caractérisé en ce qu'**on utilise le fer en tant que citrate de fer.

7. Procédé selon l'une des revendications 1 à 6, **caractérisé en ce qu'**on réalise la culture en continu en retenant les cellules à l'aide de membranes.

8. Procédé selon l'une des revendications 1 à 7, **caractérisé en ce que** le *Tetrahymena* dépendant des lipides est le *Tetrahymena setosa*.

9. Procédé selon la revendication 8, **caractérisé en ce qu'**on utilise en tant que *Tetrahymenae* dépendants des lipides le *Tetrahymena setosa* ATCC 30782, ses mutants ou ses variantes.

10. Procédé selon- l'une des revendications 1 à 7, **caractérisé** en ce le *Tetrahymena* dépendant des lipides est le *Tetrahymena rostrata*.

11. Procédé selon la revendication 10, **caractérisé** en ce le *Tetrahymena* dépendant des lipides est le *Tetrahymena rostrata* ATCC 30770.

12. Procédé selon l'une des revendications 1 à 7, **caractérisé** en ce le *Tetrahymena* dépendant des lipides est le *Tetrahymena patula*.

13. Procédé selon la revendication 12, **caractérisé** en ce le *Tetrahymena* dépendant des lipides est le *Tetrahymena patula* CCAP 1630/2.

14. Procédé pour la préparation de matières intéressantes biogènes à partir de *Tetrahymena* dépendant des lipides (lipid requirer), **caractérisé en ce qu'**on cultive les *Tetrahymena* dépendants des lipides à l'aide d'un procédé selon l'une des revendications 1 à 13 et on isole les matières biogènes intéressantes selon des méthodes connues.

15. Procédé selon la revendication 14 pour la préparation d'enzymes, que l'on a obtenues par sécrétion à partir de *Tetrahymenae* dépendants des lipides (lipid requirer) dans le milieu environnant, **caractérisé en ce qu'**on cultive les *Tetrahymenae* dépendants des lipides à l'aide d'un procédé selon l'une des revendications 1 à 13 jusqu'à l'enrichissement du milieu de culture en les enzymes sécrétées, et ensuite on isole les enzymes selon des procédés connus à partir du surnageant exempt de cellules et on les sépare à l'aide de procédés chromatographiques.

16. Procédé selon la revendication 14 pour la préparation d'enzymes associées aux cellules à partir de *Tetrahymenae* dépendants de lipides (lipid requirer), **caractérisé en ce qu'**on cultive les *Tetrahymenae* dépendants de lipides à l'aide d'un procédé selon l'une des revendications 1 à 13, on homogénéise les cellules, on sépare la solution renfermant les enzymes par centrifugation ou filtration des cellules rompues et on sépare les enzymes à l'aide de procédés chromatographiques.

17. Procédé selon la revendication 14, **caractérisé en ce que** les matières biogènes sont des acides gras plusieurs fois insaturés.
